# EUROPEAN PATENT APPLICATION

(11) **EP 0 558 857 A1**
(43) Date of publication of application: **08.09.1993**
(21) Application number: 92400500.2
(22) Date of filing: 26.02.1992
(51) Int. Cl.: C07C 209/74

(54) **Method for the manufacture of P-bromodimethyl aniline**

(71) Applicant: IMI (TAMI) INSTITUTE FOR RESEARCH & DEVELOPMENT LTD., IL-Haifa 26111 (IL)
(72) Inventor: Eisenstadt, Amihai, Ramat-Hasharon (IL); Hermolin, Joshua, Ramat-Hasharon (IL)
(74) Representative: Gillard, Marie-Louise

(57) **Abstract**

Para-bromodimethyl aniline of a high purity at high yields is obtained by the direct bromination of N,N'-dimethyl aniline with bromine in the presence of a solvent. The temperature which prevails during the reaction may be up to the reflux of the solvent used. The solvent consists of at least one halogenated hydrocarbon having the formula RXₙ wherein: R is a straight or branched aliphatic hydrocarbon having 1 to 12 carbon atoms, X is bromine or chlorine and n is between 1 and 4, with the proviso that when n is 4, the solvent is CCl₄.

## Description

The present invention relates to a simple method for the bromination of substituted aniline. More particularly, the invention relates to a simple method for the manufacture of p-bromodimethyl aniline.

### BACKGROUND OF THE INVENTION

P-bromodimethylaniline is a valuable starting material in various organic syntheses. Whereas dimethylaniline is considered a sensitive aromatic compound, the concept of the known methods for its bromination was based on the use of mild brominating agents such as: inorganic bromide and a mineral acid, N-bromosuccinimide, dibromomalonatonitrile, 2,4-dibromo-5,5-dimethylhydantoin, etc. in the presence or absence of a solvent. However, these brominating agents give unsatisfactory results concerning impurities, the product also comprising other brominated compounds such as 2-bromo- and 2,4-dibromodimethylaniline. Also these reagents are quite expensive.

In a review on kinetics of the bromination of some aromatic amines which appeared already in 1958 (R.P. Bell et al. J. of Chem. Soc. p. 161-7), there is mentioned the formation of a complex between bromine and amines. A spectrophotometric study was carried out on the reaction between bromine and p-bromodimethylaniline in sulfuric acid (0.5 M) and it was concluded that at the beginning, the bromine reacted with the amine and formed a complex and as a result normal bromination was greatly retarded. An efficient bromination for electrophilic aromatic compounds is described by Killop et al (J. Org. Chem. Vol. 37 No. 1, 1972 p. 88-92) being based on a mixture of thallium (III) acetate and bromine. It is explained that in a first stage thallium acetate is transformed into thallium (III) bromide with simultaneous formation of the brominated aromatic compound. Dimethyl aniline is also mentioned among the aromatic compounds suitable for this method of bromination.

One of the main problems encountered in the direct bromination of electrophilic aromatic compounds is the relatively large number of products resulting therefrom. Thus, in the case of direct bromination of dimethylaniline six different reaction products were mentioned (Chemical Abstracts , 1907, Vol. 1, 58).
1. Br₂C₆H₅N(CH₃)₂.
2. Br₂C₆H₅N(CH₃)₂Br₂.
3. Br₂C₆H₄N(CH₃)₂Br₂.
4. C₆H₄BrN(CH₃)HBr.
5. C₆H₄BrN(CH₃)₂HBr₃.
6. C₆H₃Br₂N(CH₃)₂HBr₃.

According to C.A., 1966, 64, 3442, p-bromodimethylaniline is obtained by the bromination of dimethylaniline using hexabromo-trimethylene trisulfone as brominating agent in a solvent such as benzene.

According to C.A., 1987, 134172n, the bromination of dimethylaniline is carried out with dibromoisocyanuric acid; a mixture of bromination products is mentioned as a result.

The above review clearly indicates that although the bromination of dimethylaniline was investigated quite extensively, no simple solution was found to be economically feasible and suggested for its direct bromination.

It is an object of the present invention to provide a simple method for the manufacture of p-bromodimethylaniline. It is another object of the present invention to provide a simple method for the direct bromination of dimethylaniline at high yields. It is yet another object of the present invention to provide a simple method for the manufacture of p-bromodimethylaniline which is substantially free of other brominated products.

### BRIEF DESCRIPTION OF THE INVENTION

The invention relates to a method for the manufacture of p-bromodimethylaniline by the direct bromination of N,N-dimethylaniline with bromine in the presence of a solvent consisting of at least one halogenated hydrocarbon having the formula: RXₙ, wherein R is the radical of a straight or branched aliphatic hydrocarbon having 1 to 12 carbon atoms; X is bromine or chlorine and n is 1 to 4 with the proviso that when n is 4 the solvent is CCl₄, said reaction being carried out at a temperature up to the reflux of the solvent. It was unexpectedly found that this bromination, yields a quantitative product with a very high selectivity of above 98%.

### BRIEF DESCRIPTION OF THE FIGURES.

Figure 1 illustrates the chromatogram of the product obtained in Example 1.

Figure 2 illustrates the chromatogram of the product obtained in Example 3.

Figure 3 illustrates the chromatogram of the product obtained in Example 4.

Figure 4 illustrates the chromatogram of the product obtained in Example 6.

Figure 5 illustrates the chromatogram of the product obtained in Example 7.

### DETAILED DESCRIPTION OF THE INVENTION.

In the solvent of formula RXₙ used in the invention, the halogen atoms X are at the end of the chain.

Typical examples of halogenated solvents useful in the present invention are: ethylene dichloride, methylene chloride, dibromomethane, etc. The aliphatic hydrocarbon bound to the halogen may be a straight or branched, saturated hydrocarbon possessing between 1-12 carbon atoms, being stable at the reaction conditions. Of course, the low alkyl hydrocarbons having between 1 and 6 carbon atoms are preferred. The most preferred solvent is methylene chloride which, due to its low boiling point can be easily removed yielding a substantially colourless and pure product.

It was most surprisingly found according to the present invention, that in the presence of the halogenated hydrocarbon, it is possible to change the orientation of the powerfully ortho-para directing amino group in aniline which could lead to the formation of substantial amounts of ortho-bromodimethyl aniline among other brominated anilines. As known, the separation of the ortho - isomer from the para - is very difficult. The inventors assume that in the presence of a halogenated hydrocarbon, the reaction system enables the electron donating characteristic of the amino group to remain in force throughout the reaction. Of course this is only an assumption of the mechanism and other elements might be involved.

It was unexpectedly found that the desired results - high yields and pure products - are obtained only when the solvent consists of a halogenated aliphatic hydrocarbon containing at least two halogens. As will appear from Examples 6 and 7, when the solvent used was chlorobenzene only poor results were obtained.

The temperature at which the direct bromination takes place may be selected in a broad range up to that of the boiling point of the solvent used. Preferred temperatures for obtaining high yields are in the range of 0 to 30°C.

The reaction time depends on the temperature utilized as well as the time of bromine addition. Of course when the reaction is carried out at low temperatures, in the range of 3 to 8°C, longer reaction times will be required than in the case when reflux temperature is utilized. However, in the latter case some side-reaction products will also be obtained. In this case a further purification might be required using known methods such as trituration with methanol or by crystallization from a methanol solution.

According to a preferred embodiment, the bromine is added slowly into the reactor under vigorous stirring. In this manner the product will be substantially free of any reaction by-products.

Among the main advantages of the method according to the present invention it should be mentioned that no recycling of the brominating agent is required as used in the known indirect methods. Whereas the method consists of a direct bromination, yielding quantitative bromination, addition of further bromine will lead to the formation of 2,4-dibromodimethyl aniline.

The entire method is very simple and requires common equipment and reagents. The reagents dimethylaniline and the halogenated solvent (at least two halogens), are introduced into a reactor equipped with a stirrer, thermometer and an opening for the introduction of bromine. At the end of the bromination, an alkaline solution is slowly added to the stirred solution and the organic layer separated. The halogenated solvent is distilled out leaving the final product. When a very high purity grade is required, the product may be treated with methanol and filtered out, or crystallized from a hot methanol solution.

Summing up, the novel method according to the present invention provides a direct bromination with high rates of conversion and high purity of the products. Accordingly, the product obtained generally does not require any additional purification step.

While the invention will now be described in connection with certain preferred embodiments in the following Examples it will be understood that it is not intended to limit the invention to these particular embodiments. On the contrary, it is intended to cover all alternatives, modifications and equivalents as may be included within the scope of the invention as defined by the appended claims. Thus the following Examples which include preferred embodiments will serve to illustrate the practice of this invention, it being understood that the particulars described are by way of example and for purposes of illustrative discussion of preferred embodiments of the present invention only.

It should be pointed out that Examples 6 and 7 do not illustrate the invention and are presented only for comparison purpose.

The gas chromatography analyses were carried out in a HP 5890A instrument using a SE 30 capillary containing 100% dimethyl siloxane.

In the Examples the percentages given are by weight unless otherwise stated.

### EXAMPLE 1.

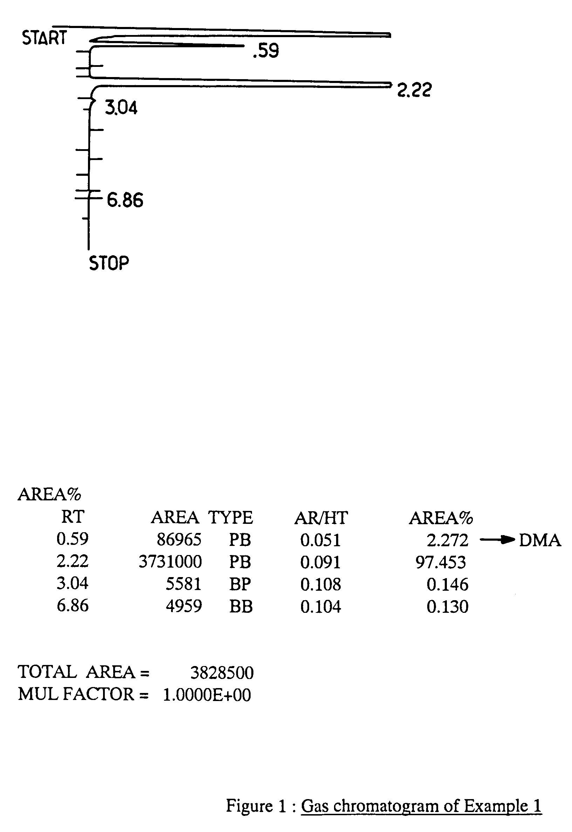

51 ml. (49 g) of N,N-dimethylaniline (0.4 moles), and 500 ml. dichloromethane were introduced into a flask of 1 litre equipped with a stirrer, thermometer and addition funnel. An amount of 63.9 g (0.4 moles) bromine was slowly added into the reaction mixture at 4-5°C whilst maintaining vigorous stirring. The bromine addition took about 4 hours. At the end of the reaction 29 g NaOH in 120 ml. water were slowly added to the stirred solution and the organic layer was separated. The dichloromethane was distilled out leaving 79.22 g (98.5%) of a white to grey solid of p-bromo-dimethylaniline of high purity (99%). The chromatogram of the product is given in Figure 1.

### EXAMPLE 2.

Example 1 was repeated except that 61 g (0.502 moles) of dimethylaniline were used and the addition of the bromine was carried out at room temperature (22-27°C). The analysis of the final product showed: 0.05% starting material, 98.9% product and 0.95% dibromo-dimethylaniline.

### EXAMPLE 3.

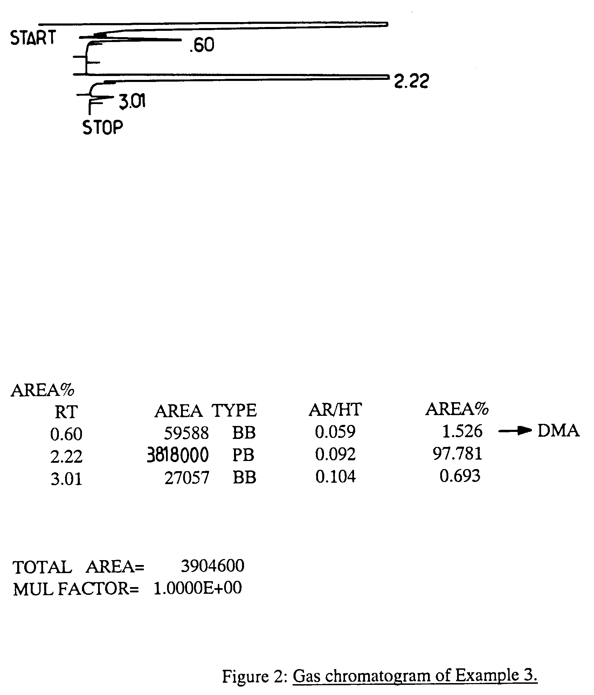

Example 1 was repeated at the boiling temperature of dichloromethane (43°C) instead of at 4-5°C, and the bromine addition took about 1.5 hours. The recovered solid product (100 g), was analysed as 97.8% P-bromo-dimethylaniline. The chromatogram of the product is given in Figure 2.

### EXAMPLE 4.

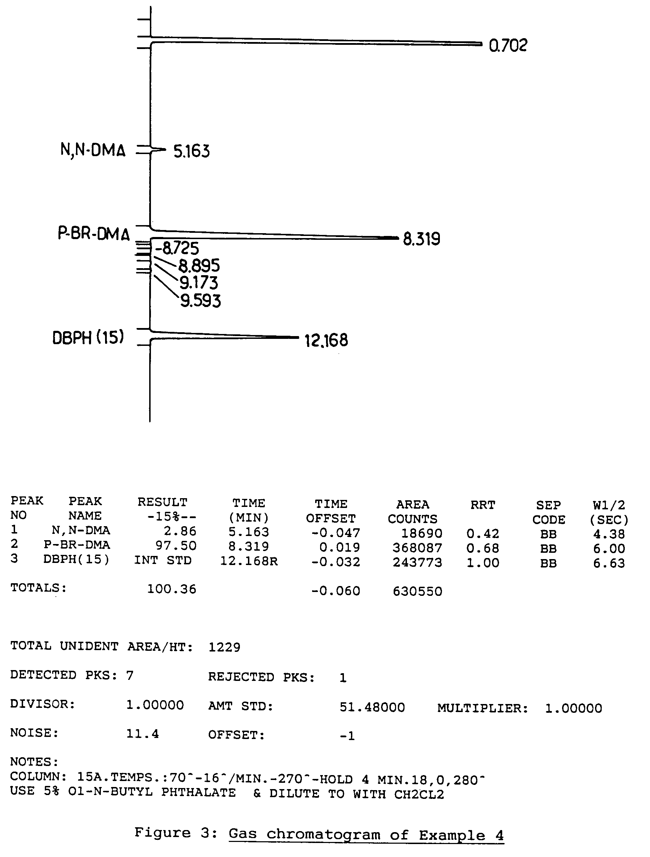

Example 2 was repeated except that an amount of 500 ml of ethylene dibromide was used instead of dichloromethane and the bromine addition took about 1.5 hr. The product obtained had the same composition as in Example 2. The chromatogram of the product is given in Figure 3.

### EXAMPLE 5.

Example 2 was repeated except that an amount of 500 ml ethylene chloride was used as a solvent instead of dichloromethane. The bromine addition took about 1.5 hours. The product obtained had the same composition as in Example 2.

### EXAMPLE 6. (not according to the invention).

Example 2 was repeated using the same amounts of reagents but the solvent used was chlorobenzene instead of dichloromethane and the temperature reached 40°C. After addition of 80% of the theoretically amount of bromine the analysis of the reaction mixture was as follows:
- 75.8 % p-bromo-dimethylaniline.
- 18.5 % dimethyl aniline.
- 5.12% dibromodimethyl aniline.

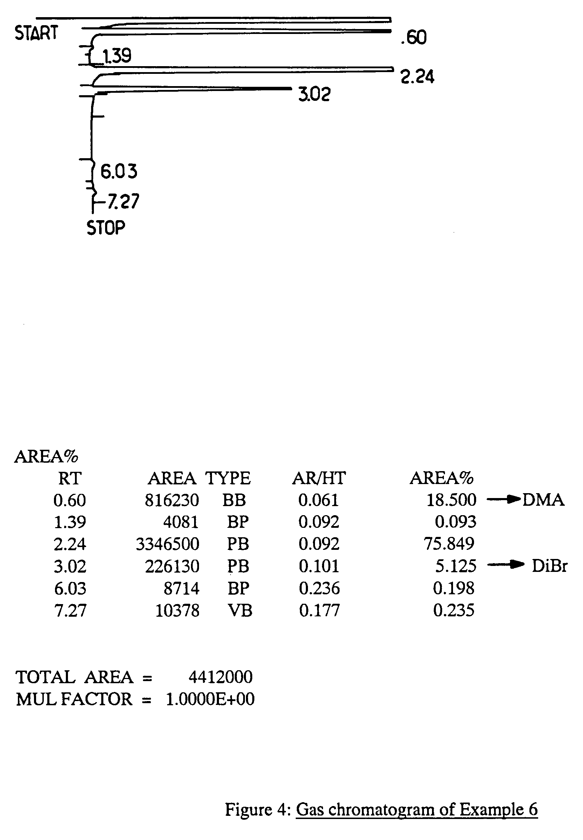

The chromatogram of the product is given in Figure 4.

### EXAMPLE 7. (not according to the invention).

Example 6 was repeated but the temperature was kept below 10°C. The final product had the following composition:
- 90.74% p-bromodimethyl aniline.
- 3.9 % dimethyl aniline.
- 4.81% dibromodimethyl aniline.

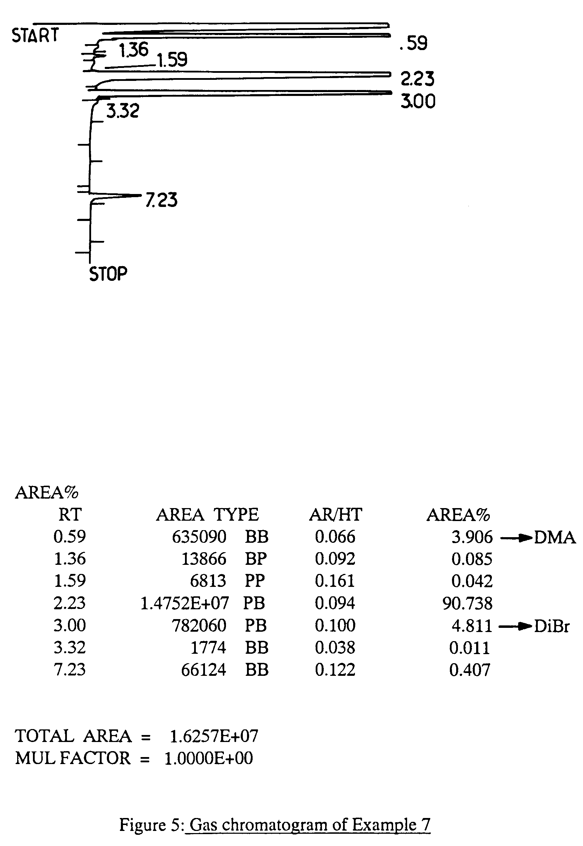

The chromatogram of the product is given in Figure 5.

## Claims

1. A method for the manufacture of p-bromodimethylaniline by the direct bromination of N,N-dimethyl-aniline with bromine in the presence of a solvent consisting of at least one halogenated hydrocarbon, stable at the reaction conditions, having the formula RXₙ, wherein
R is the radical of a straight or branched aliphatic hydrocarbon having 1 to 12 carbon atoms,
X is bromine or chlorine, and
n is 1 to 4, with the proviso that when n is 4 the solvent is CCl₄,
said reaction being carried out at a temperature up to the reflux of the solvent.

2. The method according to claim 1, wherein the reaction is carried out at a temperature in the range of 0 to 30°C.

3. The method according to claims 1 and 2, wherein the solvent used is methylene chloride.

4. The method according to claims 1 and 2, wherein the solvent used is ethylene dichloride.

5. The method according to claims 1 and 2, wherein the solvent used is dibromomethane.

6. The method according to claims 1 to 5, wherein the bromine is added slowly under a vigorous stirring.
